# EUROPEAN PATENT SPECIFICATION

(11) **EP 2 421 813 B1**
(45) Date of publication and mention of the grant of the patent: **27.03.2013**
(21) Application number: 10715789.3
(22) Date of filing: 19.04.2010
(51) Int. Cl.: A01N 25/30, C07C 305/10, C07C 41/03, C07C 43/11

(54) **METHOD FOR PREPARATION OF AND COMPOSITIONS OF LOW FOAM, NON-GELLING, SURFACTANTS**
VERFAHREN ZUR HERSTELLUNG VON UND ZUSAMMENSETZUNGEN MIT GERINGER SCHAUMBILDUNG, NICHT-GELBILDEND, TENSIDE
PROCÉDÉ DE PRÉPARATION ET COMPOSITIONS D'AGENTS TENSIOACTIFS NON COAGULANTS À FAIBLE TENEUR EN MOUSSE

(30) Priority: 22.04.2009 US 171508 P; 03.06.2009 EP 09161812
(43) Date of publication of application: 29.02.2012
(73) Proprietor: Akzo Nobel Chemicals International B.V., 3811 MH Amersfoort (NL)
(72) Inventor: NGUYEN, Giao Vinh, Friendswood TX 77546 (US)
(74) Representative: De Vries, Adriaan Jacobus
(86) International application number: PCT/EP2010/055080
(87) International publication number: WO 2010/121975

(56) References cited:
- WO-A-2006/104883
- US-A- 2 870 220
- US-A- 4 579 992
- US-A- 5 053 556
- US-A1- 2006 008 850
- US-A1- 2006 069 220
- US-B1- 6 346 509
- DATABASE BEILSTEIN BEILSTEIN INSTITUTE FOR ORGANIC CHEMISTRY, FRANKFURT-MAIN, DE; 1965, XP002548981 Database accession no. BRN2254831 & GURVICH ET AL: ZHURNAL ORGANICHESKOI KHIMII, vol. 1, 1965, pages 492-494,
- DATABASE BEILSTEIN BEILSTEIN INSTITUTE FOR ORGANIC CHEMISTRY, FRANKFURT-MAIN, DE; 1956, XP002548982 Database accession no. BRN1799632 & CHAKHOVSKOY ET AL: BULLETIN DES SOCIETES CHIMIQUES BELGES, vol. 65, 1956, pages 453-470,
- DATABASE BEILSTEIN BEILSTEIN INSTITUTE FOR ORGANIC CHEMISTRY, FRANKFURT-MAIN, DE; 1978, XP002548983 Database accession no. BRN1713350 & POMOGAILO ET AL: J.GEN.CHEM.USSR, vol. 48, 1978, pages 1911-1921,
- DATABASE BEILSTEIN BEILSTEIN INSTITUTE FOR ORGANIC CHEMISTRY, FRANKFURT-MAIN, DE; 1957, XP002548984 Database accession no. BRN1715367 & GINGRAS ET AL: CANADIAN JOURNAL OF CHEMISTRY, vol. 35, 1957, pages 599-603,
- DATABASE BEILSTEIN BEILSTEIN INSTITUTE FOR ORGANIC CHEMISTRY, FRANKFURT-MAIN, DE; 1957, XP002548985 Database accession no. BRN1801568 & GINGRAS: CANADIAN JOURNAL OF CHEMISTRY, vol. 35, 1957, pages 599-603,
- DATABASE BEILSTEIN BEILSTEIN INSTITUTE FOR ORGANIC CHEMISTRY, FRANKFURT-MAIN, DE; 1977, XP002548986 Database accession no. BRN2186937 & SHINODA ET AL: JOURNAL OF PHYSICAL CHEMISTRY, vol. 81, 1977, page 1842,
- DATABASE BEILSTEIN BEILSTEIN INSTITUTE FOR ORGANIC CHEMISTRY, FRANKFURT-MAIN, DE; 1973, XP002548987 Database accession no. BRN1915608 & HATO ET AL: JOURNAL OF PHYSICAL CHEMISTRY, vol. 77, 1973, pages 378-381,

## Description

### FIELD OF THE INVENTION

The invention relates to low foam, non-gelling, non-ionic alcohol ethoxylate surfactants and anionic ether sulfate surfactants formed therefrom, and their use in agricultural formulations.

### BACKGROUND OF THE INVENTION

Generally, non-ionic surfactants are used in a wide variety of applications. For use, for example, in agricultural formulations, non-ionic surfactants may be prepared from the alkoxylation of a hydroxyl compound, such as a fatty alcohol. The alkoxylation, which is actually the polymerization of the alkylene oxide, is normally catalyzed, or initiated, by a strong base, such as sodium or potassium hydroxide.

One conventional approach includes hydroxide-catalyzed ethoxylation, which is a polymerization reaction in which the chain growth (propagation) is much faster than the chain transfer (termination). Consequently, the conventional alcohol ethoxylate with low to moderate degree of ethoxylation (e.g. prepared from ethoxylation of the fatty alcohol with 6 moles of ethylene oxide (EO) or less) contains an excessively high concentration of the starting (unreacted) alcohol. Since the alcohol is not a surfactant, its presence in the resulting alcohol ethoxylate product is not only detrimental to the performance of the alcohol ethoxylate but also imparts hazardous properties, such as skin and eye irritation, to the ethoxylated product. Similarly, alkyl ether sulfate made from the fatty alcohol ethoxylates contains a high concentration of alkyl sulfate which, as comparable to the desired ether sulfate, is also more hydrophobic and irritating.

While providing adequate surfactancy, conventional fatty alcohol-based non-ionic surfactants (fatty alcohol ethoxylates) and their anonic alkyl ether sulfate derivatives also suffer drawbacks that make their use in certain applications undesirable. For instance, both the fatty alcohol ethoxylates and their alkyl ether sulfate derivatives undesirably gel upon contact with water. Thus, these types of conventional surfactants are more difficult to dissolve and more difficult to be rinsed off. In addition to the problems regarding solubility and rinsability, gelling also makes it difficult to produce the alkyl ether sulfates at high concentration. In addition, conventional fatty alcohol ethoxylates and their alkyl ether sulfate derivatives foam excessively. Such high foaming can prevent highly concentrated formulated products containing these ether sulfate derivatives from being diluted to their proper application dosages. Thus, there is a need for improved non-ionic surfactants and their ether sulfate derivatives that overcome the aforementioned problems.

### SUMMARY OF THE INVENTION

In a first aspect, the present invention generally relates to a two step process for the preparation of non-ionic surfactants, specifically, alcohol ethoxylates of the general structure (I):

R-(OCH₂CH₂)n-OH (I)

In a second aspect, the invention relates to alcohol ethoxylates of formula (I) made from the aforementioned process, wherein said alcohol ethoxylates have a narrow distribution range, low free alcohol content, and low ether/diacetal content.

In a third aspect, the invention relates to provide a process for preparing a low foaming, non-gelling anionic surfactant having the general formula (II):

R-(OCH₂CH₂)n-OSO₃M (II)

In a fourth aspect, the invention relates to alkyl ether sulfates of formula (II), which possess low foam, non-gelling properties while retaining their surfactancy and penetrating power.

In a fifth aspect, the present invention relates to the use of alkyl ether sulphates of formula (III) as adjutants in agriculturally active formulations.

These and further aspects of the present invention will be described more in detail in the following detailed description of the present invention.

Is is to be noted that the present invention relates to all possible combinations of the appended claims.

### BRIEF DESCRIPTION OF THE DRAWING

The invention is best understood from the following detailed description when read in connection with the accompanying drawings. Included in the drawings are the following figures:
Figure 1 is a graph depicting the distribution of non-ionic surfactant made from the ethoxylation of2-ethyl hexanol (2EH) with 4 moles of ethylene oxide using the regular, KOH-catalyzed process and the two stage process of this invention.
Figure 2 is a graph illustrating the ethylene oxide distribution of 2-ethylhexanol (2EH) + 4 moles of ethylene oxide (4EO) for three samples made by different ethoxylation processes.
Figure 3 is a depiction of the effect of low non-ionic on clarity of an ether sulfate in accordance with an embodiment of the present invention compared to that of a conventional ether sulfate.

### DETAILED DESCRIPTION OF THE INVENTION

### (A) Two Stage Process for the Preparation of Alcohol Ethoxylates and Narrow Range Products Formed Therefrom

In a first embodiment, the present invention generally relates to a two stage process for the preparation of a low foaming, non-gelling, non-ionic alcohol alkoxylate surfactant, specifically, alcohol ethoxylates of the general structure (I):

R-(OCH₂CH₂)n-OH (I)

where R is an alkyl group containing 6-11 carbon atoms and n, which represents the number of ethylene oxide units contained in the hydrophilic group of the nonionic surfactant structure, is an integer of from 3- 6. The process is a two stage process whereby in a first stage, (i) the ethoxylation of starting fatty alcohol is catalyzed with a suitable Lewis Acid, for e.g., boron trifluoride (BF₃) to form an alcohol ethoxylate precursor, followed by (ii) further ethoxylation of the resulting alcohol ethoxylate precursor is catalyzed by a strong base, for e.g., potassium hydroxide. The temperature in stage (i) of the above process is typically in the range of from 90 to 130°C. The temperature in stage (ii) of the above process is typically in the range of from 100 to 130°C.

Prior art processes are generally single step catalyzed with, for example, a Lewis Acid, such as BF₃, heavy alkaline, such as barium hydroxide, or an Al-Mg hydroxide complex, such as hydrotalcite. These processes, however, are not preferred for the preparation of the desired non-ionic surfactants because, for example, a high degree of ethoxylation with BF₃ as the catalyst results in extensive formation of dioxane, a hazardous by-product. Additionally, the toxicity of barium hydroxide renders the resulting non-ionic product hazardous and thus unsuitable for most applications. Finally, the fine particle size of the hydrotalcite-type catalyst makes its removal from the desired non-ionic product extremely difficult.

The claimed invention overcomes many of the deficiencies of the prior art by providing a process for preparing non-ionic surfactants using a two-stage alkoxylation process. In the first stage, a first amount of ethylene oxide reacted with a fatty alcohol in an ethoxylation reaction in the presence of a suitable Lewis acid ethoxylation catalyst, e.g., BF₃ or complexes thereof to form an alcohol ethoxylate precursor. The first stage provides a narrow distribution base for the product while minimizing the formation of undesired by-products. A small dosage of BF₃, for example, about 0.05% to about 0.07% by weight of the final product (the alcohol ethoxylate of formula (I)), is used as the catalyst. BF₃ Etherate, a complex of BF₃ with diethyl ether (BF3:OEt₂) is also an effective catalyst for the ethoxylation and is preferred over the gaseous BF₃ since it is less hazardous and easier to handle than the gaseous BF₃. Typically, BF₃ Etherate is also used at small dosage, about 0.10 - 0.17% by weight of the final product. Other complexes of BF₃ with oxygen containing donors, such as complexes with dibutyl ether (BF₃:OBu₂), tetrahydrofuran (BF₃:THF), methanol (BF₃: MeOH), phosphoric acid (BF₃:H₃PO₄), acetic acid (BF₃:AcOH) and the like, can also be used at concentration of about 0.10 - 0.25% by weight of the final products. Boron trichloride (BCl₃) and halides of other Group III elements such as aluminum trichloride (AlCl₃) can also be used as catalysts for the alkoxylation in this stage; however, in general, they are weaker Lewis Acids, and require larger dosage to be comparable to BF₃ in catalytic effectiveness. The quantity of ethylene oxide used in this step varies from 25% to 75% of the total ethylene oxide used for the preparation of the final product. It is important to note that several by-products are formed in this stage, and not all of them are undesired. For example, while dioxane and acetal, the most common by-products, are undesired due to their hazardous property, dialkyl poly(oxyethyl) ether (III) is preferred since it is non-hazardous and could serve as the "surfactancy booster" to enhance the surfactancy of the non-ionic products of this invention and of their ether sulfate derivatives.

R'-x(OCH₂CH₂)-O-(CH₂CH₂O)_{y}-R' (III)

Thus, the quantity of ethylene oxide used in this stage is regulated to control the concentrate of the "surfactancy booster" in the final non-ionic product as needed to obtain its optimum performance in the designed application. For example, in certain embodiments, up to 2 moles of EO per mole of fatty alcohol (R-OH) could be used in this stage. In a preferred embodiment, the non-ionic surfactant resulting from the inventive process will include less than 8%, and even more preferably less than 5%, of the starting, unreacted alcohol.

The second stage allows the degree of alkoxylation to reach the desired level while maintaining the narrow distribution established from the first stage and prevents further formation of by-product. In this second stage, a strong base, such as sodium hydroxide or potassium hydroxide, is employed as a catalyst at a significant dosage, for example, of from 0.5% to 0.7% by weight of the final product. In the preparation of alcohol ethoxylates according to this embodiment, the second stage involves reacting the remaining quantity of ethylene oxide needed to make a final product in an ethoxylation reaction with the fatty alcohol precursor in the presence of the base catalyst.

In other embodiments of the present invention, the preparation of the final non-ionic surfactant product involves the use of a mixed oxide. In such embodiments, the process involves the ethoxylation of ethylene oxide with the fatty alcohol precursor and then reacting the ethoxylation product with another alkylene oxide, such as propylene oxide. In still further embodiments of the present invention, the two stages of the process could also be reversible, with the second stage base-catalyzed alkoxylation preceding the catalyzed ethoxylation.

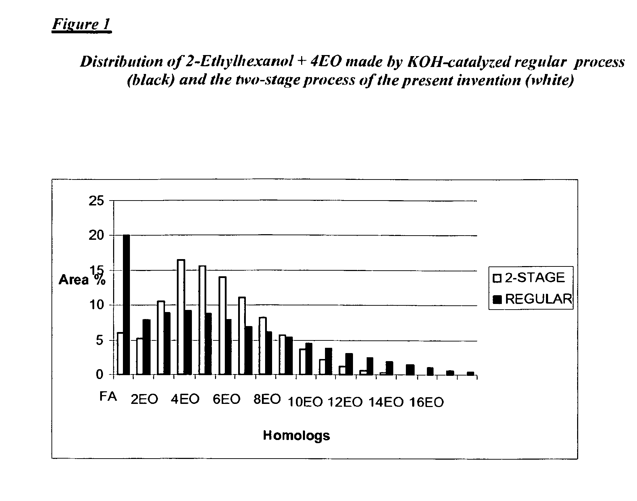

The present invention also provides advantages over conventional alcohol ethoxylates made by conventional KOH-catalyzed ethoxylation processes. For example, even when proper starting alcohol and proper degree of ethoxylation are employed, the resulting alcohol ethoxylates made by the common KOH-catalyzed ethoxylation process do not function properly as surfactants. Materials made by this process possess wide distributions. Figure 1 illustrates the distribution of an exemplary non-ionic surfactant made from the ethoxylation of 2-ethylhexanol (2EH) with 4 moles of ethylene oxide (4EO) by the regular KOH-catalyzed process and the two stage process of this invention.

As shown in Figure 1, the final alcohol ethoxylate made by the regular process contains up to 20% of the starting 2EH, which does not possess surfactancy. It also includes up to 20% of alcohol ethoxylate homologs with more than 8 units on the polyethylene oxide (PEO) chain, which are undesirably too hydrophilic. Consequently, only 60% of the product mixture possesses the desired degree of ethoxylation, i.e. 1 - 8 EO units in the POE chain, and thus the desired surfactancy and hydrophilicity. In comparison, the final alcohol ethoxylate of the present invention has much less starting (unreacted) alcohol (6 wt%), less alcohol ethoxylate homologs with more than 8 units on the polyethylene oxide (PEO) chain, and much narrower distribution which up to 86% of the product mixtures possesses the desired degree of ethoxylation, i.e. 1 - 8EO units in the POE chain.

The invention also relates to alcohol ethoxylates of formula (I) made from the aforementioned process, wherein said alcohol ethoxylates have a narrow distribution range, low free alcohol content, and low ether/diacetal content.

R-(OCH₂CH₂)n-OH (I)

where R is a straight, branched or cyclic, saturated or unsaturated alkyl group containing from 6 - 11 carbon atoms, and n, which represents the number of ethylene oxide units contained in the hydrophilic group of the nonionic surfactant structure, is an integer of from 3 - 6. In another embodiment, R is a branched alkyl group of from 6 to 9 carbon atoms. To provide optimum performance in most applications, the alcohol ethoxylates with lower degree of ethoxylation (i.e., made with less than 6 moles of EO) should contain less free (unreacted) alcohol and less by-products, and should possess peaked distribution in which the sum of the concentration of the five most abundant PEO homologs is more than 60% of the weight of the alcohol ethoxylate. Typically, conventional alcohol ethoxylates made by the KOH-catalyzed ethoxylation with 4 moles ofEO contains an insignificant amount (less than 2%) of by-products. However, their distribution is flat (the sum of the concentration of the five most abundant homologs is less than 45% of the weight of the alcohol ethoxylates), and their free alcohol content is excessively high (up to 20 %). In contrast, conventional alcohol ethoxylates made by the Lewis Acid catalyzed ethoxylation with the same number of moles of EO possess narrow (peaked) distribution and contains very low level of the free alcohol (less than about 3%). However, their content of by-products (up to about 15%) is high. The alcohol ethoxylates of this invention made from the same number of moles of EO offer the desired properties of both types of conventional alcohol ethoxylates. Compared to conventional alcohol ethoxylates made by the regular KOH-catalyzed process, the alcohol ethoxylates of the present invention have a narrow distribution range and low free alcohol content. Compared to conventional alcohol ethoxylates made by the single stage ethoxylation catalyzed by the Lewis Acid such as BF₃, the alcohol ethoxylates of the present invention have a comparable narrow distribution range and low free alcohol content while simultaneously possessing low content of by-products. Typically, alcohol ethoxylates of this invention made with 4-5 moles of EO possess a peaked distribution with the sum of the concentration of the five most abundant homologs of over 60%, and contain less than 8% of free alcohol and less than 4% of total by-products.

To possess low foam, non-gelling properties while retaining their surfactancy, embodiments of the invention include at least about 80% of alcohol ethoxylate homologs of the non-ionic surfactant that preferably have from 1 to 2n ethylene oxide units in their hydrophilic group. Further, embodiments of the invention also include non-ionic surfactants that include less than about 15% of alcohol ethoxylate homologs of the non-ionic surfactant that have more than 2n ethylene oxide units in their hydrophilic group.

To obtain the desired low foam, non-gelling characteristics of the non-ionic surfactant of the present invention, the chain length of the starting alcohol should be 11 carbons or less. However, surfactancy is reduced by decreasing the alcohol chain length. Preferably, the chain length of the alcohol is in the range of from 6 - 10 carbons atoms, and more preferably of from 7 - 9 carbon atoms. Although linear alcohols may be used, they tend to generate more foam and gel more than their branched counterparts. Suitably starting alcohols for the preparation of the low foaming, non-gelling non-ionic surfactants include, but are not limited to 2-ethylhexanol (2EH, branched alcohol with 8 carbons , CH₃CH₂CH₂CH₂CH(CH₂CH₃)CH₂OH), and 2-propylheptanol (2PH, branched alcohol with 10 carbons,
CH₃CH₂CH₂CH₂CH₂CH(CH2CH₂CH₃)CH₂OH) are preferred. Other suitable starting alcohols include 1-hexanol (CH₃CH₂CH₂CH₂CH₂CH₂OH), 2-hexanol
(CH₃CH₂CH₂CH₂CH₂CH(OH)CH₃₎, 1-heptanol (CH₃CH₂CH₂CH₂CH₂CH₂CH₂OH), 2-heptanol (CH₃CH₂CH₂CH₂CH₂CH(OH)CH₃₎, 1-octanol (CH₃CH₂CH₂CH₂CH₂CH₂CH₂CH₂OH), cyclohexanol ((CH₂)₅CHOH), 2-methylcyclohexanol ((CH₂)₄CH(CH₃)CHOH), 4-tert-butylcyclohexanol (CH₂)₂CH(C(CH₃)₃)(CH₂)₂CHOH), 4-methoxycyclohexanol ((CH₂)₂CH(OCH₃)(CH₂)₂CHOH) and combinations and mixtures thereof.

In addition to the chain length of the starting alcohol, the degree ofethoxylation also affects extent of the low foam, non-gelling characteristics of the non-ionic surfactant. Specifically, attaching a chain of several ethylene oxide units to the alcohol provides hydrophilicity and thus completes the structure of the non-ionic surfactant. The chain length of the polyethylene oxide (PEO) constituent, which is controlled by the degree of ethoxylation, strongly affects the surfactancy of the resulting alcohol ethoxylates. Generally, a high degree of ethoxylation results in a too hydrophilic product. Similarly, low degree of ethoxylation results in a too hydrophobic group. Preferably, in an embodiment of the present invention, in the structure of the non-ionic surfactants the starting alcohol is ethoxylated with at least 3 moles ofethylene oxide and/or up to and including 5 moles of ethylene oxide.

In another embodiment of the present invention, the non-ionic surfactant may further include a "surfactancy booster." A surfactancy booster enhances the surfactant properties, such as surface tension reduction or wetting capability, of the non-ionic surfactants. Suitable surfactancy boosters may include both anionic and non-ionic surfactants. However, the addition of such surfactancy boosters may result in the undesired increase in foaming and/or gelling of the latter. Preferable surfactancy boosters for use in the present invention are dialkyl poly(oxyethyl) ethers, such as those having the following general structure (III):

R'-x(OCH₂CH₂)-O-(CH₂CH₂O)y-R' (III)

where R' is an alkyl group containing 4-12 carbon atoms , x is 1- 10 and y is 0 - 10. These materials can be prepared from the condensation of the non-ionic surfactants of the present invention or they may be formed *in situ* during the ethoxylation process. Preferably, the surfactancy booster is added in an amount of from about 1% to about 3% by weight of the nonionic surfactant.

### B). Process for Making Ether Sulfates

Ether sulfates are materials possessing the general structure (II):

R-(OCH₂CH₂)ₙ-OSO₃M (II)

Typically, ether sulfates are prepared from the sulfation of the alcohol alkoxylates or alkylphenol alkoxylates (more commonly, alcohol ethoxylates), as shown in the below formula.

R- (OCH₂CH₂)ₙ - OH + SO₃ → R - (OCH₂CH₂)ₙ - OSO₃H

Sulfation is then followed by the neutralization of the resulting ester acid with a selected base, such as sodium hydroxide or ammonium hydroxide, as shown below.

R - (OCH₂CH₂)ₙ - OSO₃H + NaOH → R - (OCH₂CH₂)ₙ - OSO₃Na

The most common ether sulfates are those based on the alcohol ethoxylates that, in turn, are prepared from the ethoxylation of mid-cut synthetic or natural alcohols with 10 - 14 carbon atoms with 1 - 3 moles of ethylene oxide. These ether sulfates are used mainly as detergents /cleaning agents in most cleaning and care applications. They are also used as adjuvants for glufosinate herbicides functioning as penetrating agents to improve the herbicides' bioefficacy via improving their adsorption into the targeted plant.

The present invention provides a process for preparing a low foaming, non-gelling anionic surfactant, specifically ether sulfates, having the general formula (II):

R-(OCH₂CH₂)ₙ-OSO₃M (II)

where R is an alkyl group containing 6-11 carbon atoms and n is an integer of from 3-6, said process comprising: (i) reacting a first amount of ethylene oxide with a fatty alcohol in the presence of a Lewis acid catalyst to form an alcohol ethoxylate precursor; (ii) reacting a second amount of ethylene oxide with said alcohol ethoxylate precursor in the presence of a base catalyst thereby forming an alcohol ethoxylate surfactant having a general structure represented by the formula (I):

R-(OCH₂CH₂)ₙ-OH (I)

where R is an alkyl group containing 6 - 11 carbon atoms and n is an integer of from 3-6; (iii) reacting said alcohol ethoxylate surfactant with sulfur trioxide to form an ester acid; and (iv) neutralizing said ester acid with a base.

In this context, the alcohol ethoxylate prepared by the aforementioned inventive process or the aforementioned alcholethoxylate, each previously mentioned as separate aspects of the present invention, here acts as an intermediate for the formation of the ether sulphates of formula (II).

The ether sulfates of the present invention are low foaming and non-gelling, readily dilutable and/or rinsable without gelling or foaming. These ether sulfates may be prepared at higher active (up to 80%) without requiring specific equipment or handling processes, and they remain stable and flowable even at room temperature.

The anionic surfactants according to the present invention are thus based on the non-ionic surfactant alcohol ethoxylate precursors as discussed above. Accordingly, the preparation of the anionic surfactant ether sulfates includes the two-stage alkoxylation process, which, as set forth previously, may also be reversed. The first stage provides the narrow distribution base for the product while minimizing the formation of undesired by-products, for example dioxane, PEG, acetal and ether. The second stage provides the degree of ethoxylation to reach the desired level while maintaining the narrow distribution established from the first step and prevents further formation of by-product. In addition, the process results in significant reduction of viscosity of the high active ether sulfate derivative, and thus makes its production using available neutralization equipment more practical.

The process for preparing the low foaming, non-gelling anionic surfactant according to embodiments of the present invention further comprises the steps of reacting the alcohol ethoxylate surfactant with sulfur trioxide to form an ester acid and neutralizing said ester acid with a base. Generally, these steps may include conventional equipment and processes of making low active ether sulfates that are known to those of ordinary skill in the art. An example of such a process includes the continuous sulfation of the alcohol ethoxylate precursor with sulfur trioxide diluted with air (air-SO₃) on a falling film reactor. The sulfation is followed by the continuous neutralization of the resulting ester acid with the selected base in the selected solvent, for example, water. Other substances, including light alcohols such as ethanol, isopropanol, butanol, glycols such as ethylene glycol, propylene glycol, and glycol ethers, such as dipropylene glycol methyl ether, propylene glycol methyl ether, ethylene glycol butyl ether, can be used as solvent, or as co-solvent in combination with water to modify or adjust the physical properties of the final ether sulfates.

In another embodiment, the invention relates to an alkyl ether sulfate of the formula (II):

R-(OCH₂CH₂)ₙ-OSO₃M (II)

where R is a straight, branched or cyclic chain, saturated or unsaturated alkyl group containing 6 - 11 carbon atoms and n is an integer of from 3- 6. In another embodiment, R is a branched alkyl group.

The ether sulfates of the present invention preferably comprise at least about 75% solids and up to and including about 25% water. To possess low foam, non-gelling properties while retaining their surfactancy and penetrating power, embodiments of ether sulfates in accordance with the invention preferably include, based on its solids, at least about 80%, and more preferably at least about 85% of alkyl ether sulfate homologs having from 1 to 2n ethylene oxide units in their hydrophilic group. Further, embodiments of the invention also include anionic surfactants comprising ether sulfates that include less than about 15%, and more preferably less than about 10% of alkyl ether sulfate homologs that have more than 2n ethylene oxide units in their hydrophilic group. The ether sulfates of the present invention also preferably include less than about 8%, and preferably less than about 5% of the non-ethoxylate alkyl sulfate.

To obtain the desired low foam, non-gelling characteristics of the desired ether sulfates of the present invention, the chain length of the starting alcohol should be 10 carbons or less. However, as surfactancy of the final ether sulfates is reduced by decreasing the alcohol chain length, the chain length of the alcohol is preferably in the range of from 6-10 carbons atoms, and more preferably of from 7 - 9 carbon atoms. Although linear alcohols may be used, these tend to foam and gel more so than their branched counterparts. Among the suitable starting alcohols for the preparation of the ether sulfates in accordance with the present invention, 2-ethylhexanol (2EH, branched alcohol with 8 carbons) and 2-propylheptanol (2PH, branched alcohol with 10 carbons) are preferred.

In addition to the chain length of the starting alcohol, the degree ofethoxylation also affects the surfactancy of the final ether sulfate. Specifically, attaching a chain of several ethylene oxide units to the precursor alcohol ethoxylate provides the hydrophilicity for the alcohol ethoxylate and thus the ether sulfate derivative. The chain length of the polyethylene oxide (PEO), which is controlled by the degree of ethoxylation, strongly affects the surfactancy of the final ether sulfate. Generally, a high degree of ethoxylation results in improved solubility but lower surfactancy. Likewise, a low degree of ethoxylation enhances surfactancy but lowers the solubility. Preferably, in an embodiment of the present invention, the structure of the anionic surfactant ether sulfates includes a starting alcohol of 2EH or 2PH ethoxylated with at least 3 moles of ethylene oxide and/or up to and including 5 moles of ethylene oxide.

In comparison, for example, with conventional ether sulfates prepared by conventional KOH-catalyzed ethoxylation, the ether sulfates of the present invention provide a much higher degree of ethoxylation. The KOH-catalyzed ethoxylation of, for example, 2EH with 4EO results in only 60% of the alcohol ethoxylate precursor having the desired degree ofethoxylation (1-8 EO units in the POE chain), only 60% of the final ether sulfate species will also possess the desired degree of ethoxylation. To obtain the desired surfactancy and penetrating power, the concentration of these species in the ether sulfate must be 85% or higher of the ether sulfate solids.

In embodiments of the present invention, the ether sulfates are prepared as salts, which allow the ether sulfates to be stable in the aqueous medium. Among the suitable salts for use in the present invention, sodium and ammonium salts are preferred. Other suitable salts include potassium salt, alkanol amine salts, such as triethanolamine (TEA), and light amine salts, such as isopropylamine. Such salts allow the ether sulfates of the present invention to be produced at up to and including about 80% active while still remaining stable and flowable. Other suitable salts include sodium or potassium, although these are less preferred. These salt versions of the ether sulfates may be prepared at up to about 70% active without the need for specific equipment or special handling.

In further embodiments of the present invention, the anionic surfactant may further include a "surfactancy booster." A surfactancy booster enhances the surfactant properties, such as surface tension reduction or wetting capability, of the final ether sulfates. Suitable surfactancy boosters may include both anionic and non-ionic surfactants. However, the addition of such surfactancy boosters may result in the undesired increase in foaming and/or gelling of the latter. Preferable, surfactancy boosters for use in the present invention are dialkyl poly(oxyethyl) ethers, such as those having the general structure (III), as set forth above. These materials can be prepared from the condensation of the precursor alcohol ethoxylate of the ether sulfates of the present invention or they may be formed *in situ* during the ethoxylation process in which the precursor alcohol ethoxylates are prepared. Preferably, the surfactancy booster is added in an amount of from about 1% to about 3% by weight of the anionic surfactant. If the amount surfactancy booster exceeds about 4%, separation of the ether sulfate and an undesirably high viscosity may result.

The ether sulfates of the present invention may be used in a variety of applications. One suitable application is in agricultural formulations in which the ether sulfates are mixed with certain agriculturally active ingredients. For example, the ether sulfates may be mixed with water-soluble agriculturally active ingredients, such as glufosinate (salts), glyphosate (salts), paraquat, diquat and the like, and in particular glufosinate-ammonium. Suitable active ingredients can include those which, during preparation of aqueous spray liquors, are fully or partially dissolved in the aqueous phase, generally dissolved by 1 to 100 percent by weight, preferably by 5 to 100 percent by weight, more preferably by 10 to 100 percent by weight, in particular by 20 to 100 percent by weight, very particularly preferably by 30 to 100 percent by weight, based on the weight of the active ingredient in the spray liquor, preferably at the active ingredient concentrations as are customary in art. Examples of such active ingredients are, in addition to the active crop protectant ingredients mentioned above, those selected from among herbicides, fungicides, insecticides, acaricides, anthelmintics and other active ingredients, such as plant growth regulators and safeners, preferably active ingredients such as, for example, iodosulfuron-methyl-sodium, imidacloprid, thiacloprid, prothioconazole and triadimenol.

Exemplary active ingredients may also include insoluble active ingredients from classes of substances which nominally include active ingredients of different solubilities, examples being active ingredients from the group of the cyclohexanedione derivatives, imidazolinones, pyrimidyloxypyridinecarboxylic acid derivatives, pyrimidyloxybenzoic acid derivatives, sulfonylureas, triazolopyrimidinesulfonamide derivatives, and S-(N-aryl-N-alkylcarbamoylmethyl)d ithiophosphoric esters.

Further suitable agricultural active ingredients include partially water-soluble fertilizers, preferably foliar fertilizers (fertilizers which are taken up by the leaves of the plants), such as urea or foliar macro- or microelement fertilizer, including chelates. Also suitable is the combination of active crop protectant ingredients, such as herbicides, insecticides, fungicides, plant growth regulators and, if desired, fertilizers as agricultural active ingredients. In exemplary embodiments of the present invention, the ether sulfates are used to replace conventional ether sulfates, such as lauryl ether sulfate, that are included as adjuvants in concentrated glufosinate herbicide formulations.

Such agriculturally active ingredients that are suitable for use in combination with the ether sulfates of the present invention are further described in U.S. Patent Application No. 11/765,039, published as U.S. Publication No. 2008/0045415 on February 21, 2008.

### EXAMPLES

Examples of the preparation of the desired hybrid alcohol ethoxylate precursor and high-active ether sulfate based on the hybrid alcohol ethoxylate precursor are provided below.

### A, Preparation of Alcohol Ethoxylate

In a first stage, a 1003g sample of 2-ethylhexanol (EH) was charged to a one-gallon alkoxylation reactor and dehydrated at 115-120°C under a slight nitrogen purge until the moisture content was less than 0.05%. After the dehydrated alcohol was cooled to about 90°C, 3g of boron trifluoride-etherate, included as a catalyst, was charged to the reactor. The alcohol-catalyst mixture was then mixed for 10 minutes, then purged with nitrogen 5 times to remove air and heated to 105°C. Next, 684g of ethylene oxide (EO) was added to the reactor. An exothermic reaction occurred immediately and cooling was applied throughout the EO addition to maintain the reaction temperature within the range of 105-115°C. Following the EO addition, the reaction mixture was digested at 115°C for one hour then purged with nitrogen five times and cooled to 90°C.

In a second stage, 31 g of 45% potassium hydroxide, included as a second catalyst, was charged to the reactor. The mixture was then mixed for 10 minutes and then dehydrated at 110°C under nitrogen purge. When the moisture content of the mixture was less than 0.1% (after about 120 minutes of dehydration) 665g of EO was then added to the reactor. Here again, an exothermic reaction occurred and cooling was applied throughout the EO addition to maintain the reaction temperature within the range of 120-130°C. Following the EO addition, the ethoxylation reaction mixture was digested at 125°C-130°C for one hour, then purged with nitrogen five times and cooled to 65°C. Next, 16g of acetic acid was added and then the product mixture was mixed for 15 minutes then discharged. About 2270g of alcohol ethoxylate was collected.

The resulting 2EH + 4EO alcohol ethoxylate (Sample 3) was analyzed and compared with its analogs made by a conventional potassium hydroxide (KOH)-catalyzed ethoxylation process (Sample 1) and by a conventional boron trifluoride-catalyzed ethoxylation process (Sample 2). To determine the degree of ethoxylation via OH value, wet analysis was used. Gas chromatography (GC) was used to determine EO distribution and liquid chromatography-mass spectrometry (LC/MS) and nuclear magnetic resonance (NMR) were used for a determination of unwanted by-products. The results of these analyses are summarized in Table 1.

**Table 1: Properties of Ethoxylated 2-Ethylhexanol**

| | **Sample 1** | **Sample 2** | **Sample 3** |
|---|---|---|---|
| Ethoxylation process | Regular (KOH) | Narrow Range (BF₃) | Two Stage (Hybrid) |
| Appearance at 25°C | Clear liquid | Clear liquid | Clear liquid |
| OH Number, mg KOH/g | 186.0 | 188.6 | 186.3 |
| Moisture, wt% | < 0.10 | < 0.1 | < 0.1 |
| Diacetal + POE Ether, wt% | < 0.10 | 11.2 | 1.2 |

The results identified in Table 1 confirm the desired effect of the inventive process on the formation of by-product. By using only 50% of the total quantity of EO in the Lewis Acid catalyzed ethoxylation stage and thus cutting the EO/Alcohol molar ratio from 4:1 (Narrow Range Process) to 2:1 (Two Stage Process), the content of by-products (e.g. diacetal and ether) in the final ethoxylate product is reduced by over 90%.

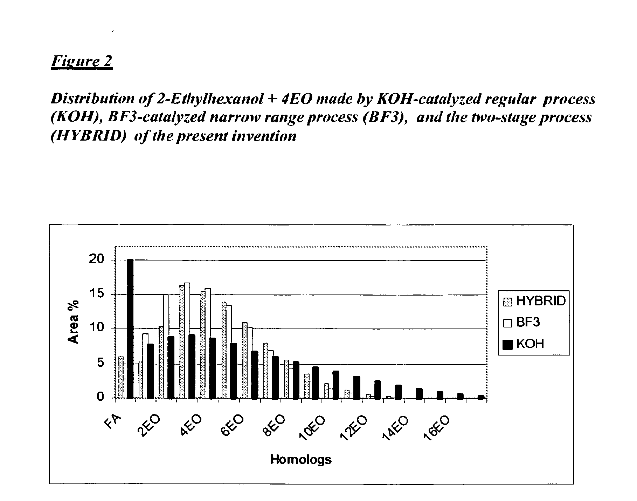

In addition, Figure 2 illustrates the EO distribution of the three samples reported in Table 1. As shown in Figure 2, the sample of the alcohol ethoxylate made by the process according to embodiments of the present invention, i.e. Sample 3 contains much less free alcohol than compared to Sample 1, which is an analog made by the regular KOH-catalyzed process. As compared to the alcohol ethoxylate made by the BF3-catalyzed narrow range process, i.e. Sample 2, Sample 3 possesses virtually the same narrow range distribution.

### B. Preparation of Ether Sulfate

The following describes an example of the preparation of a final ether sulfate according to an embodiment of the present invention.

### i. Preparation of the Ester Acid Intermediate

The ester acid intermediate was made by a batch sulfation process using a 2-liter multi-necked flask equipped with a mechanical stirrer, a gas sparger, a cool water bath, and a scrubber.

In preparing the ester acid intermediate, 850g of alcohol ethoxylate (850g) prepared in accordance with the two-stage process described in Example A was charged to the flask and stirred. Next, an air-SO₃ gas stream containing about 3.5 wt% of sulfur trioxide in dried air was drawn from commercial gas plant and introduced under the liquid via the gas sparger. Because the sulfation was highly exothermic, the cool water bath was used throughout the reaction to maintain the temperature of the reaction mixture in the range of 38 - 45°C. When the acid number of the reaction mixture was within the desired range, i.e. 140 - 150 mg KOH/g, addition of air-SO₃ was stopped. Nitrogen purge was then applied sub-surface for 15 - 20 minutes to remove the sulfur trioxide (SO₃) trapped in the acid product. About 1050g of ester acid was produced. Results of the analysis indicated the following: Acid Number = 147.6 mg KOH/g, Active = 2.497 me/g, Free Sulfuric Acid = 0.65%.

### ii. Neutralization of the Ester Acid Intermediate

The neutralization the ester acid to yield the 70% ether sulfate was performed in a 2-liter multi-necked flask equipped with a pH meter, and a water batch. Mixing of the neutralization materials was provided by a high-torque mechanical stirrer with speed varied from 250 to about 500 rpm.

In neutralizing the ester acid intermediate, 160g of a 50% sodium hydroxide solution, 160g of water and 1 g of a 50% citric acid solution were charged to the flask and stirred. A total of 754g of the preserved ester acid precursor was then charged slowly and steadily to the neutralizer. The neutralization was highly exothermic, thus the ice-water bath was used during the addition of the first half of the acid to maintain the temperature at less than 55°C. The addition of the ester acid was stopped when the pH of the neutralization mixture (pH = 9.5 @ 5% aqueous) was within the desired range of 9.0 - 10.5. The batch was then mixed for 30 minutes to allow the gelling ester acid (if any) to be dissolved and neutralized. Ultimately, about 1030g of the final ether sulfate was discharged.

### C. Properties of Final Ether Sulfate

The properties of the final ether sulfate, identified as ES 1, made from the alcohol ethoxylate in accordance with an embodiment of the present invention, are summarized in Table 2 below. For comparison purposes, properties of a high active analogous ether sulfate based on alcohol ethoxylate made by the conventional narrow range ethoxylation process, identified as ES 2, are also identified in Table 2.

**Table 2: Properties of 70% Ether Sulfate**

| Property | ES 1 | ES 2 |
|---|---|---|
| Appearance | Slightly hazy viscous liquid | Highly viscous slurry |
| Total solids, wt% | 73.3 | 75.1 |
| Moisture, wt% | 26.7 | 25.6 |
| Sodium sulfate, wt% | 0.6 | 0.3 |
| Nonionic, wt% | 0.5 | 2.7 |
| Active (by difference), wt% | 72.2 | 72.1 |
| pH (5% aqueous) | 9.8 | 10.0 |

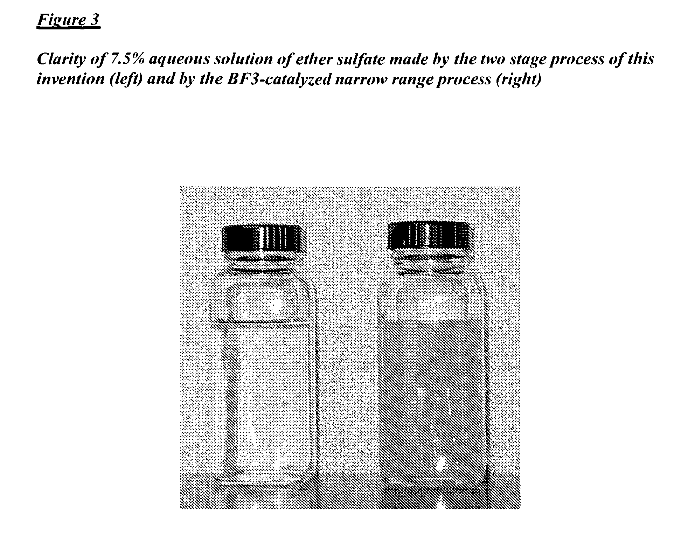

As shown above, the two sample materials have similar properties; however, sample ES 1 possesses much lower free non-ionic. Not wishing to be bound by theory, it is possible that the effect of sample ES 1's lower free nonionic content is manifested by the resulting lower viscosity and associated slight haziness, as compared to sample ES 2. The effect of free non-ionic on viscosity is beneficial in that additional capital equipment, such as a high torque mixer for neutralization, is not required. The effect of low non-ionic on clarity of the final ether sulfate is also significant. As shown in Figure 3, 7.5% active solution of sample ES 2 turned turbid after only 15 minutes of standing, while the 7.5% active solution of sample ES remained clear after several days of standing.

## Claims

1. A process for preparing an alcohol alkoxylate of the formula:
R-(OCH₂CH₂)n-OH (I)
where R is an alkyl group containing 6-11 carbon atoms and n is an integer of from 3 - 6, said process comprising:
reacting, at a temperature of from 90 to 130°C, a first amount of ethylene oxide with a fatty alcohol of formula R-OH in the presence of a Lewis Acid catalyst to form an alcohol ethoxylate precursor; and
reacting, at a temperature of from 100 to 130°C, a second amount of ethylene oxide with said alcohol ethoxylate precursor in the presence of a base catalyst.

2. The process of claim 1 Wherein said R is a branched alkyl group of 6-9 carbon atoms.

3. The process of claim 1 or 2 wherein the molar ratio of ethylene oxide to fatty alcohol used in the Lewis Acid-catalyzed stage is up to 2:1.

4. The process of any of the preceding claims wherein said Lewis acid catalyst is boron trifluoride or a complex thereof

5. The process of any of the preceding claims, further comprising adding a surfactancy booster during reacting step (ii).

6. The process of claim 5 wherein said surfactancy booster comprises a dialkyl poly(oxyethyl) ether having a general structure represented by the formula (III):
R'-x(OCH₂CH₂)-O-(CH₂CH₂O)y-R' (III)
wherein R' is C4 - C12, x is 1 - 10 and y is 0 - 10.

7. The process of any of the preceding claims, wherein said first amount of ethylene oxide is from 25% to 75% of the total ethylene oxide used in the process, and the second amount of ethylene oxide is the balance of the total ethylene oxide used in the process.

8. A process for preparing an alkyl ether sulfate of formula (II):
R-(OCH₂CH₂)ₙ-OSO₃M (II)
where R is a straight, branched or cyclic chain, saturated or unsaturated alkyl group containing 6 - 11 carbon atoms and n is an integer of from 3 - 6, said process comprising:
performing the process of any of the claims 1 to 7 to provide an alcohol ethoxylate of formula (I)
R-(OCH₂CH₂)n-OH (I)
reacting said alcohol ethoxylate with sulfur trioxide to form an ester acid; and neutralizing said ester acid with a base.

9. The process of claim 8 wherein said base is selected from the group consisting of an ammonium salt, an alkanol amine salt, a light amine salt, a sodium salt and a potassium salt.

## Patentansprüche

1. Ein Verfahren zur Herstellung eines Alkoholalkoxylats der Formel:
R-(OCH₂CH₂)n-OH (I)
wobei R ein Alkylrest mit 6-11 Kohlenstoffatomen ist und n eine ganze Zahl von 3-6 ist, wobei das Verfahren umfasst:
Umsetzen, bei einer Temperatur von 90 bis 130°C, einer ersten Menge Ethylenoxid mit einem Fettalkohol der Formel R-OH in Gegenwart eines Lewissäure-Katalysators, um einen Alkoholethoxylat-Vorläufer zu bilden; und
Umsetzen, bei einer Temperatur von 100 bis 130°C, einer zweiten Menge Ethylenoxid mit dem Alkoholethoxylat-Vorläufer in Gegenwart eines basischen Katalysators.

2. Das Verfahren nach Anspruch 1, wobei das R ein verzweigter Alkylrest mit 6-9 Kohlenstoffatomen ist.

3. Das Verfahren nach Anspruch 1 oder 2, wobei das in der Lewissäure-katalysierten Stufe verwendete Molverhältnis von Ethylenoxid zu Fettalkohol bis zu 2:1 beträgt.

4. Das Verfahren nach einem der vorhergehenden Ansprüche, wobei der Lewissäure-Katalysator Bortrifluorid oder ein Komplex davon ist.

5. Das Verfahren nach einem der vorhergehenden Ansprüche, weiter umfassend Zugabe eines die Grenzflächenaktivität verstärkenden Mittels während Reaktionsschritt (ii).

6. Das Verfahren nach Anspruch 5, wobei das die Grenzflächenaktivität verstärkende Mittel einen Dialkylpoly(oxyethyl)ether mit einer allgemeinen Struktur, dargestellt durch die Formel (III), umfasst:
R'—x(OCH₂CH₂)-O-(CH₂CH₂O)y-R' (III)
wobei R' C4-C12 ist, x 1-10 ist und y 0-10 ist.

7. Das Verfahren nach einem der vorhergehenden Ansprüche, wobei die erste Menge Ethylenoxid 25% bis 75% des gesamten in dem Verfahren verwendeten Ethylenoxids beträgt und die zweite Menge Ethylenoxid den Rest des gesamten in dem Verfahren verwendeten Ethylenoxids ausmacht.

8. Ein Verfahren zur Herstellung eines Alkylethersulfats der Formel (II):
R-(OCH₂CH₂)n-OSO₃M (II)
wobei R ein geradkettiger, verzweigter oder cyclischer, gesättigter oder ungesättigter Alkylrest mit 6-11 Kohlenstoffatomen ist und n eine ganze Zahl von 3-6 ist, wobei das Verfahren umfasst:
Durchführen des Verfahrens nach einem der Ansprüche 1 bis 7, um ein Alkoholethoxylat der Formel (I) bereitzustellen
R-(OCH₂CH₂)n-OH (II)
Umsetzen des Alkoholethoxylats mit Schwefeltrioxid, um eine Estersäure zu bilden; und Neutralisieren der Estersäure mit einer Base.

9. Das Verfahren nach Anspruch 8, wobei die Base aus der Gruppe bestehend aus einem Ammoniumsalz, einem Alkanolaminsalz, einem leichten Aminsalz, einem Natriumsalz und einem Kaliumsalz ausgewählt ist.

## Revendications

1. Procédé de préparation d'un alcoxylat d'alcool de formule :
R—(OCH₂CH₂)n-OH (I)
où R est un groupe alkyle contenant 6 à 11 atomes de carbone et n est un nombre entier de 3 à 6, ledit procédé comprenant les étapes consistant à :
faire réagir, à une température de 90 à 130 °C, une première quantité d'oxyde d'éthylène avec un alcool gras de formule R-OH en présence d'un catalyseur de type acide de Lewis pour former un précurseur d'éthoxylat d'alcool ; et
faire réagir, à une température de 100 à 130 °C, une seconde quantité d'oxyde d'éthylène avec ledit précurseur d'éthoxylat d'alcool en présence d'un catalyseur de type base.

2. Procédé selon la revendication 1, dans lequel ledit R est un groupe alkyle ramifié de 6 à 9 atomes de carbone.

3. Procédé selon la revendication 1 ou 2, dans lequel le rapport molaire entre oxyde d'éthylène et alcool gras utilisés dans l'étape catalysé par acide de Lewis va jusqu'à 2 : 1.

4. Procédé selon l'une quelconque des revendications précédentes, dans lequel ledit catalyseur de type acide de Lewis est le trifluorure de bore ou un complexe de celui-ci.

5. Procédé selon l'une quelconque des revendications précédentes, comprenant en outre l'étape consistant à ajouter un intensificateur de propriétés tensioactives pendant l'étape (ii) de réaction.

6. Procédé selon la revendication 5, dans lequel ledit intensificateur de propriétés tensioactives comprend un dialkyl poly(oxyéthyl)éther ayant une structure générale représentée par la formule (III) :
R'-x(OCH₂CH₂)-O-(CH₂CH₂O)y-R' (III)
dans laquelle R' est en C₄ à C₁₂, x vaut 1 à 10 et y vaut 0 à 10.

7. Procédé selon l'une quelconque des revendications précédentes, dans lequel la première quantité d'oxyde d'éthylène est de 25 % à 75 % de l'oxyde d'éthylène total utilisé dans le procédé, et la seconde quantité d'oxyde d'éthylène est le reste de l'oxyde d'éthylène total utilisé dans le procédé.

8. Procédé de préparation d'un alkyléther sulfate de formule (II) :
R-(OCH₂CH₂)n-OSO₃M (II)
où R est un groupe alkyle saturé ou insaturé à chaîne droite, ramifiée ou cyclique contenant 6 à 11 atomes de carbone et n est un nombre entier de 3 à 6, ledit procédé comprenant les étapes consistant à :
réaliser le procédé de l'une quelconque des revendications 1 à 7 pour former un éthoxylat d'alcool de formule (I)
R-(OCH₂CH₂)n-OH (I)
faire réagir ledit éthoxylat d'alcool avec du trioxyde de soufre pour former un acide ester ; et
neutraliser ledit acide ester avec une base.

9. Procédé selon la revendication 8, dans lequel ladite base est choisie dans le groupe consistant en un sel d'ammonium, un sel d'alcanolamine, un sel d'amine légère, un sel de sodium et un sel de potassium.
